# EUROPÄISCHE PATENTSCHRIFT

(11) **EP 2 754 386 B1**
(45) Veröffentlichungstag und Bekanntmachung des Hinweises auf die Patenterteilung: **20.07.2016**
(21) Anmeldenummer: 13006031.2
(22) Anmeldetag: 20.12.2013
(51) Int. Cl.: A61B 3/09, A61B 5/00, A61F 2/16, A61B 5/04, A61B 5/0488, A61B 5/0492, A61B 5/0496, A61B 5/11

(54) **Sensorsystem für die Erfassung der Ansteuersignale eines Ziliarmuskels**
Sensor system for detecting the activation signals of a ciliary muscle
Système de capteurs pour l'acquisition des signaux de commande d'un muscle ciliaire

(30) Priorität: 10.01.2013 DE 102013000429
(43) Veröffentlichungstag der Anmeldung: 16.07.2014
(73) Patentinhaber: Karlsruher Institut Für Technologie (KIT), 76131 Karlsruhe (DE)
(72) Erfinder: Guth, Helmut, 76344 Eggenstein-Leopoldshafen (DE); Gengenbach, Ulrich, 75196 Remchingen (DE); Bretthauer, Georg, 76139 Karlsruhe (DE); Fliedner, Jan, 76131 Karlsruhe (DE); Beck, Christoph, 76139 Karlsruhe (DE); Krug, Markus, 76133 Karlsruhe (DE); Martin, Thomas, 76139 Karlsruhe (DE); Nagel, Jörg, 76344 Eggenstein-Leopoldshafen (DE); Koker, Liane, 76297 Stutensee (DE); Sieber, Ingo, 76133 Karlsruhe (DE)

(56) Entgegenhaltungen:
- EP-A1- 1 726 272
- WO-A1-2010/004094
- WO-A1-2012/006691
- DE-A1- 10 155 345
- US-A1- 2006 122 530
- US-A1- 2010 331 977

## Beschreibung

Die Erfindung betrifft ein Sensorsystem für die Erfassung der Ansteuersingale eines Ziliarmuskels eines Auges gemäß dem ersten Patentanspruch. Das Sensorsystem umfasst ein Auswertesystem vorzugsweise für die Bestimmung des Akkommodationsbedarfs für eine künstliche Linse vorzugsweise eines künstlichen Akkommodationssystems oder eines ophthalmischen technischen Systems.

Das menschliche Auge ist ein natürliches optisches System, das Objekte mit Hilfe mehrerer lichtbrechender Grenzflächen scharf auf der Netzhaut (retina) abbildet. Ändert sich die Gegenstandsweite des betrachteten Objektes, ist es für eine Abbildung mit gleich bleibender Schärfe auf der Netzhaut notwendig, das sich das Abbildungsverhalten des optischen Systems ändert. Beim menschlichen Auge wird dies durch eine Verformung der Linse mit Hilfe des Ziliarmuskels (musculus ciliaris) realisiert, wodurch sich im Wesentlichen die Form und die Lage der Linsenvorder- und -rückseite ändern (Akkommodation).

Das menschliche Auge verliert im Alter die Fähigkeit zur Akkommodation, d.h. die Anpassungsfähigkeit des natürlichen Augenlinsensystems an unterschiedliche Brennweiten. Diese wird im gesunden Auge dadurch realisiert, dass mit der vorgenannten Verformung der Linse durch den Ziliarmuskels der Krümmungsradius der Linseoberflächen und damit ihre Brechkraft variiert werden. Im Laufe des Lebens versteift die natürliche Linse und kann deshalb ihren Krümmungsradius nicht mehr in ausreichendem Maße an verschiedene Fokuslängen anpassen. Dieser Verlust muss durch Sehhilfen kompensiert werden, der Mensch leidet an Presbyopie.

Auch wenn mit fortschreitendem Alter die menschliche Augenlinse versteift und dadurch keine Akkommodation mehr möglich ist, bleibt der Ziliarmuskel weiter aktiv. In [1] wird ein geringer Einfluss des Alters auf die Ziliarmuskelaktivität beschrieben, wobei die Aktivität mit zunehmendem Alter sinkt, jedoch nie vollständig erlischt. Davon unabhängig sind die Ansteuersignale des Ziliarmuskels, die auch mit einem fortgeschrittenen Alter sich nicht nennenswert abschwächen.

Ein weiterer Grund für den Verlust der Akkommodationsfähigkeit ist die Katarakt, auch grauer Star genannt. Bei dieser Krankheit trübt die natürliche Linse ein, wodurch der Mensch erblinden kann. Derzeit wird zur Behandlung eine künstliche Intraokularlinse mit einer festen Brennweiteneinstellung implantiert, die die Transparenz wiederherstellt. Aufgrund ihres unveränderlichen Krümmungsradius ist jedoch keine Akkommodation des Auges mehr möglich, obwohl der Ziliarmuskel weiterhin seine Ansteuersignale erhalten.

Mit dem Ziel, eine mangelnde Akkommodationsfähigkeit im natürlichen Auge auszugleichen, sind verschiedene Ansätze für akkommodierende ophthalmische technische Systeme bekannt. Neben einem künstlichen Akkommodationssystem, welches als Implantat komplett wie eine Intraokularlinse in den Kapselsack implantiert wird (vgl. **[2]),** werden auch aktiv akkommodierende Systeme als integrale Bestandteile von Kontaktlinsen oder Brillen entwickelt (vgl. **[6 - 8].** Kontaktlinsen haben im Vergleich zur Implantation im Kapselsack ein noch geringeres Volumen und weisen damit einen sehr geringen Bauraum für integrierte aktive Komponenten auf. Die Anordnung von. Kontaktlinsen auf der Kornea des Auges birgt gegenüber im Kapselsack implantierten Systemen veränderte Anforderungen und Möglichkeiten zur Realisierung der Sensoreinheit zur Erfassung des Akkommodationsbedarfs. Mit Hilfe dieser Information über den Akkommodationsbedarf kann die in der Kontaktlinse integrierte aktive Optik auf die vom Patienten benötigte Brennweite eingestellt und verändert werden.

Ein Akkommodationsbedarf eines Auges ist über die Erfassung der Ziliarmuskelaktivität möglich. Die Ansteuersignale des Ziliarmuskels sind über elektrische Aktivitäten der Muskeln z.B. mittels Potentialmessungen erfassbar.

In der Praxis eignen sich zur Messung einer Muskelaktivität insbesondere aufgesetzte oder implantierte Oberflächenelektroden oder Feindrahtelektroden, welche in den Muskel verlegt werden. Die meisten bekannten Systeme zur Messung von Muskelaktivität beschränken sich jedoch auf die Messung größerer Muskelpartien.

In **[3]** wird im Rahmen eines implantierbaren künstlichen Akkommodationssystems die Möglichkeit diskutiert, die Muskelpotentiale des Ziliarmuskels vom Kapselsack aus zu messen und damit den Akkommodationsbedarf zu ermitteln. Es wurde festgehalten, dass das messbare Potential im Kapselsack aufgrund des großen Abstandes des Implantats zum Ziliarkörper zu gering sei.

**[4]** offenbart eine Versuchseinrichtung für Potentialmessungen direkt auf der Hornhaut eines Augapfels. Hierzu sind auf einem direkt auf der Hornhaut im Bereich der Iris aufliegenden Kontaktelement vier Elektroden vorgesehen, bei der eine dieser Elektroden einen direkten Kontakt zur Hornhaut aufweist. Die Elektroden sind mit Kabeln versehen, die über das offene Auge mit einer weiteren Signalverarbeitung verbunden sind.

[5] beschreibt eine Kontaktlinse mit vier Elektroden speziell zur mehrdimensionalen Erfassung der Ziliarmuskelaktivität. Die Elektroden sind auf der Innenseite einer Kontaktlinse mit gleichem Abstand zum Kontaktlinsenmittelpunkt sowie zueinander angeordnet. Die Kontakte zu den Elektroden werden über Leiterbahnen auf der Außenseite der Kontaktlinse realisiert. Obwohl die Vorrichtung der Erforschung.der Aktivität der verschiedenen Muskelfasern des Ziliarkörpers während der Akkommodation dienen soll, erfolgt keine getrennte Erfassung von Einzelfaserrichtungen. Die Anordnung und das Messprinzip lässt keine Aussage zu, aus welchen Muskelsignalen sich das gemessene Signal zusammensetzt. Dadurch ist es z.B. nicht möglich zwischen der Aktivität der Ziliarmuskeln und der Aktivität des Irismuskels zu unterscheidet. Eine zuverlässige Bestimmung des Akkommodationsbedarfs ist damit erheblich eingeschränkt.

DE10155345 offenbart ein weiteres Sensorsystem für die Erfassung der Ansteuersignale eines Ziliarmuskels.

Davon ausgehend liegt die **Aufgabe der Erfindung** darin, ein Sensorsystem zur Erfassung der Ansteuersignale eines Ziliarmuskels eines Auges vorzuschlagen, das die Aktivitäten des Ziliarmuskels richtungsaufgelöst erfasst und sich auch für eine Erfassung des Akkommodationsbedarfs eignet.

Die Aufgabe wird mit einem Sensorsystem gemäß dem ersten Patentanspruch gelöst. Unteransprüche hierzu geben vorteilhafte Ausgestaltungen wieder.

Zur Lösung der Aufgabe dient ein Sensorsystem für die Erfassung der Ansteuersingale eines Ziliarmuskels eines Auges. Voraussetzung ist, dass der Ziliarmuskel aktiv durch die Ansteuersignale angesteuert wird, und diese auf der Hornhaut (Cornea) des Auges und nicht auf dem zum Ziliarmuskel weiter entfernten Kapselsack erfassbar sind. Im Bereich um die Iris schließt sich der Bereich des Ziliarmuskels unmittelbar an. Folglich umfasst das Sensorsystem ein auf der Cornea um die Iris eines Auges applizierbares Kontaktelement aus einem elektrisch nicht leitenden Werkstoff, auf der die Sensoren des Sensorsystems in einer ringförmigen Anordnung vorzugsweise auf einer Ringfläche um die Iris und vorzugsweise auf der zur Cornea ausgerichteten Seite aufgesetzt sind. Die Elektroden der Sensoren stehen vorzugsweise in direktem Kontakt zur Cornea; ein chirurgischer Eingriff ist nicht erforderlich.

Ist das Kontaktelement eine Kontaktlinse, lässt sich nicht nur das Sensorsystem, sondern auch ein komplettes ophthalmisches technisches System, vorzugsweise ein künstliches Akkommodationssystem mit dem genannten Sensorsystem und ein in ihrer Brennweite verstellbare künstliche Linse oder ein Linsensystem in der Kontaktlinse unterbringen (aktive Kontaktlinse). Vorzugsweise alle für einen autarken Betrieb des Akkommodationssystems erforderlichen Komponenten werden dabei auf oder in der Kontaktlinse integriert.

Ist das Kontaktelement ein Kontaktring, erstreckt es sich vorzugsweise um die Iris, ohne diese oder zumindest die Pupille nicht zu überdecken. Das Sensorsystem erfasst in dieser Ausführung nur die Ansteuersignale der Ziliarmuskel, verarbeitet diese zu einem Steuersignal um und leitet dieses vorzugsweise über entsprechende Übertragungsmittel (z.B. über elektromagnetische Wellen oder mittels Kabel) an ein eigenständiges ophthalmisches technisches System wie z.B. eine akkommodierende Brille oder ein implantierbares verstellbares Linsensystem weiter. Das Sensorsystem umfasst hierzu eine Signalverarbeitung mit Übertragungsmitteln zu einem durch das Steuersignal in seiner Brennweite aktiv verstellbaren Linsenkörper oder Linsensystem, wobei die übertragenen Signale nicht zwingend das Steuersignal umfassen.

Der Ziliarmuskel dient der Anpassung der Linsen im Auge an verschiedene Objektentfernungen. Er umfasst zwei unterschiedlich orientierte und unabhängig voneinander wirkende Muskelfasergruppen, dem sog. Müllerschen Muskel mit ringförmig um die Augenlinse, d.h. tangential zur Iris orientierten Muskelfasern und dem sog. Brückschen Muskel mit meridional, d.h. radial zur Linsenachse (radial zur Iris), d.h. zur Iris orientierten Muskelfasern. Der Müllersche Muskel ist um die Linse angeordnet und dient der radialen Kontraktion des Ziliarkörpers. Durch eine Kontraktion werden die Zonulafasern entspannt und es verändert sich die Form und Oberfläche der Linse und damit die Brennweite. Der Brücksche Muskel dient dagegen der axialen Verschiebung der körpereigenen Linsen. Der Müllersche Muskel dient insbesondere der Nahakkommodation, der Brücksche Muskel insbesondere der Korrektur von Ferneinstellungen. Ein altersbedingtes Nachlassen der Akkommodationsleistung wird nicht durch ein Nachlassen der Muskelaktivität, sondern wesentlich durch eine Versteifung der Augenlinse hervorgerufen.

Ein Muskel baut im nicht innervierten Zustand ein Ruhepotential z.B. von etwa -85 mV auf. Bei der Innervation strömen Natriumionen in die Muskelzelle hinein und Kaliumionen aus der Muskelzelle heraus. Dadurch ändert sich das Zellpotential kurzzeitig, z.B. ca. 2 ms auf z.B. 30 mV (Depolarisation), baut sich im Anschluss danach wieder ab (Repolarisation) und erreicht über einen Gegenschwinger (Hyperpolarisation) wieder das Ruhepotential. Diese Erregung verläuft in einem Zeitraum von ca. 2-4 m/s entlang eines motorischen Nervs durch den Muskel, d.h. in Muskelfaserrichtung ab. Die entstandene Potentialänderung kann gemessen und daraus auf die Muskelaktivität rückgeschlossen werden.

Ein wesentliches Merkmal der Lösung basiert auf der Möglichkeit der selektiven Erfassung der Ansteuersignale der vorgenannten durch ihre Ausrichtung um die Iris gekennzeichneten Müllerschen und Brückschen Muskel. Hierzu werden Sensoren vorgeschlagen, die mindestens drei in mindestens einer Ausrichtung in Reihe angeordnete Elektroden umfassen. Die Sensoren umfassen jeweils eine mittlere Elektrode. (Bezugselektrode) und zwei beidseitig und in Ausrichtung angrenzende Messelektroden. Während die mittlere Elektrode auf ein Bezugspotential gelegt wird, wird mit den mindestens beiden angrenzenden Messelektroden die jeweils lokal anliegenden Potentiale auf der Cornea erfasst. Diese vorzugsweise direkt auf der Cornea anliegenden Elektroden der Sensoren erfassen in vorteilhafter Weise die elektrischen Potentialunterschiede zur mittleren Elektrode (je Elektrode eine Messung) in der jeweiligen Ausrichtung selektiv. Wird eine Muskelfaser durch Anspannung erregt, ist diese durch die an den beiden Messelektroden anliegenden Potentialunterschiede nur dann erfassbar, wenn die Faserrichtung parallel zur Sensorausrichtung liegt. Eine Fehlmessung z.B. aufgrund einer Verschiebung der Elektroden von den Muskelfasern oder durch eine von der Faserrichtung abweichende Ausrichtung ist über eine größere Abweichung der in einem Sensor in einer Ausrichtung erfassten Potentialunterschiede detektierbar, die Messung durch eine nachgeschaltete Signalverarbeitung für eine Steuerung z.B. eines ophthalmischen technischen Systems eliminierbar.

In einer bevorzugten Ausführung umfasst das Sensorsystem mindestens zwei Sensoren, deren Ausrichtungen sowohl tangential und als auch radial zur Iris orientierte Ausrichtungen umfassen. Damit sind die Muskelfasern der radial zur Iris orientierten Brückschen Muskeln wie auch der tangential zur Iris orientierten Müllerschen Muskeln jeweils selektiv erfassbar.

Eine weiter verbesserte selektive Erfassung ist dadurch erzielbar, dass jeder Sensor entweder nur eine tangential oder nur eine radial zur Iris orientierte Ausrichtung aufweist, d.h. keine Sensoren mit weiteren von den vorgenannten abweichenden Ausrichtungen vorhanden sind. Vorzugsweise sind die letztgenannten Sensoren in abwechselnder Reihenfolge der Ausrichtung um die Iris aufgereiht.

Das Sensorsystem umfasst weiterhin eine Signalverarbeitung auf dem Kontaktelement. Die Sensoren sind mit der Signalverarbeitung über Sensorkontakte zur Datenübertragung in Verbindung. Sie umfasst vorzugsweise eine Signalkonditionierung, eine Signalerkennung und einen Generator für ein Steuersignal. Die Signalkonditionierung dient der Aufbereitung der Sensorrohdaten, d.h. insbesondere der Eliminierung von Störsignalen. Sie umfasst neben Verstärkerstufen insbesondere Signale manipulierende Komponenten wie Filter und Gleichrichter. Die Singalerkennung greift die aufbereiteten Sensorrohdaten auf und führt sie einer Signalauswertung zu, auf deren Basis ein Generator Steuersignale für die Sensoren einerseits und nachfolgende System wie z.B. ein technisches ophthamologisches System mit verstellbaren Linsen oder ein künstliches Akkommodationssystem andererseits erzeugt. Die Signalauswertung liefert für jeden Sensor das Aktivierungspotential (Ansteuersignal) der jeweiligen Muskelstränge.

Die Signalauswertung vergleicht und/oder verrechnet die gelieferten Sensorrohdaten zu einer Aktivierung des Ziliarmuskels (z.B. positiv für eine Nahakkommodation, hauptsächlich durch die Müllersehen Muskelfasern hervorgerufen, null für den Ruhezustand und negativ für die Aktivierung der Brückschen Muskelfasern für die Fernakkommodation). Eine Aufgabe der Signalauswertung ist damit das Unterdrücken von Störungen und das Isolieren der summativen Muskelstrangsignale für jeweils die Müllersehen und die Brückschen Muskelfaserstränge. Die Signalauswertung benötigt hierzu vorzugsweise auch alle Kalibrierungsdaten die über ein Kommunikationsinterface übermittelbar sind.

Die Erfindung erstreckt sich ferner auf eine Verwendung der vorgenannten Mittel zur Ansteuerung (Steuerung oder Auslösung) einer Akkommodationsbedarfserfassung und/oder für eine Brennweiteneinstellung für ein ophthamologisches technisches System oder ein Akkommodationssystem sowie auf ein Verfahren zur Steuerung eines ophthamologischen technischen Systems oder ein Akkommodationssystem.

Die Auswertung der Muskelsignale des Ziliarmuskels birgt alle Vorteile eines Pupillennahreflexsensors wie die Verwendung des körpereigenen Regelkreises zur natürlichen Akkommodation und die intuitive Benutzbarkeit durch den Systemträger. Störeinflüsse, wie der Lichteinfall wirken nicht auf den Ziliarmuskel, was eine wesentlich höhere Zuverlässigkeit verspricht. Die geringe Komplexität der Signalaufbereitung und Auswertung der Ziliarmuskelaktivität verspricht einen sehr geringen Leistungsbedarf für das Mess- und Auswertesystem.

Ein Pupillennahreflexsensor detektiert eine Körperreaktion, die auch die Ausgabe eines Signals eines Akkommodationsregelkreises umfassen kann. Ist ein solches Signal selektiv von den übrigen Pupillenreaktionen (z.B. Einfluss von Adrenalin, Koffein, Müdigkeit, etc.) erfassbar, ist der körpereigene Akkommödations-/Schärferegelkreis für die Erfassung des Akkommodationsbedarfs nutzbar. Ein Teil der Akkommodationsregelung übernimmt nach einer Adaptionsphase das Gehirn.

Sensoren, die beispielsweise den Vergenzwinkel zwischen den Fixierlinien der Augen auswerten, können lediglich Signale auswerten die hauptsächlich aus dem Vergenzregelkreis des menschlichen Gehirns stammen. Die Vergenzreaktion ist teilweise sehr zeitverzögert und enthält für die Sensorik störende Überschwinger. Außerdem wird die Erfassung des Vergenzwinkels durch äußere Störgrößen beeinflusst (z.B. Erschütterungen Beschleunigungssensor oder Verzerrungen im Erdmagnetfeld Magnetfeldsensor).

Eine Auswertung der Ziliarmuskelaktivität ist sehr vorteilhaft, da das System durch die Auswertung der Ziliarmuskelaktivität direkten Zugriff auf die Ausgabe des Akkommodationsregelkreises des menschlichen Gehirns erhält. Der Akkommodationsbedarf muss nicht umständlich über Umwege geschätzt werden, sondern kann direkt über das Aktivierungspotential des Ziliarmuskels ermittelt werden. Die Auswertung des Ziliarmuskels bietet damit die Möglichkeit ohne großen Rechenaufwand ein künstliches Akkommodationssystem oder eine künstliche verstellbare Optik mit einer eines körpereigenen Augenlinsensystems vergleichbarer Dynamik zu schaffen. In vorteilhafter Weise wird dem menschliche Gehirn ein Lernprozess ermöglicht, sich auf das System einzustellen. Vorzugsweise wird lediglich eine sehr einfache Basiskalibrierung durchgeführt und die Feinkalibrierung dem Lernvermögen des Gehirn überlassen.

Das Sensorsystem ist grundsätzlich für verschiedene Anwendunden einsetzbar.

Vorzugsweise ist das Sensorsystem vollständig in oder auf einer Kontaktlinse integriert. Dazu werden die beschriebenen Sensoren zusammen mit einer Signalverarbeitung und einem Energiespeicher und/oder ggf. andere Mitteln oder Quellen zur Einbringung von Energie im Randbereich der Kontaktlinse aufgebracht. Mittig in der Linse befindet sich eine aktive Optik mit variabler Brechkraft. Die Brechkraft kann nun an den vom beschriebenen Sensorsystem erfassten Akkommodationsbedarf dynamisch angepasst werden.

Alternativ umfasst das Sensorsystem ein vorzugsweise kontaktloses Übertragungsmittel als Schnittstelle zu einer externen Energiequelle und/oder einem externen akkommödierenden oder verstellbaren Linsensystem. Über diese Schnittstelle können Akkommodationsinformationen an eine akkommodierende Brille oder ein akkommodierende ophthamlomogischen System übermittelt werden. Der Vorteil von externen Komponenten wie z.B. einer Brille als adaptives optisches Element besteht darin, dass ein erheblich größerer Bauraum zur Verfügung steht. Eine solche Brille besteht aus vorzugsweise einem optischen Element mit variabler Brechkraft, einer Steuereinheit für dieses Element, eine Energieversorgung und ebenfalls Übertragungsmittel zum Sensorsystem. Das Sensorsystem wird vorzugsweise über die Brille mit Energie versorgt werden. Dadurch ist der Energiespeicher des Sensorsystems auf einen optionalen Energiepuffer reduzierbar, d.h. signifikant verkleinerbar.

Auch der Einsatz der beschrieben Sensoreinheit in Kombination mit einem aktiven implantierbaren Akkommodationssystem oder einer aktiven Intraokluarlinse (IOL) bietet Vorteile. Zum einen kann der Bauraum für ein Sensorsystem in der Bauraumkritischen IOL eingespart werden. Zum anderen werden die Nachteile der bekannten Sensorkonzepte wie z.B. Konvergenzwinkelmessung oder Pupillennahreflex zur Bestimmung des Akkommodationsbedarfs ohne Heranziehung des Ziliarmuskels eliminiert. Die IOL beinhaltet dieselben Elemente wie die vorgenannte akkommodierende Brille, d.h. optisches Element mit variabler Brechkraft, Steuereinheit, Energiespeicher/-quelle und Übertragungsmittel.

Die Erfindung und weitere Details und Ausführungsoptionen werden im Folgenden anhand von Ausführungsbeispielen und Details mit Figuren näher erläutert, die optional auch mit einzelnen oder allen vorgenannten Maßnahmen zusätzlich kombinierbar oder erweiterbar sind. Es zeigen
**Fig.1a** **und b** schematische Darstellungen beispielhafter Ausführungen eines Sensors des Sensorsystems,
**Fig.2** einen Teilquerschnitt durch das Auge mit applizierten Sensorsystem im Bereich der Pupille mit Ziliarmuskel und Sensorsystem,
**Fig. 3a** **bis f** schematische Darstellungen der Ausführungen des Sensorsystems mit Elektroden und Ringelelektrode sowie
**Fig.4** eine schematische Systemschaltskizze eines Sensorsystem mit Sensor und Signalverarbeitung.

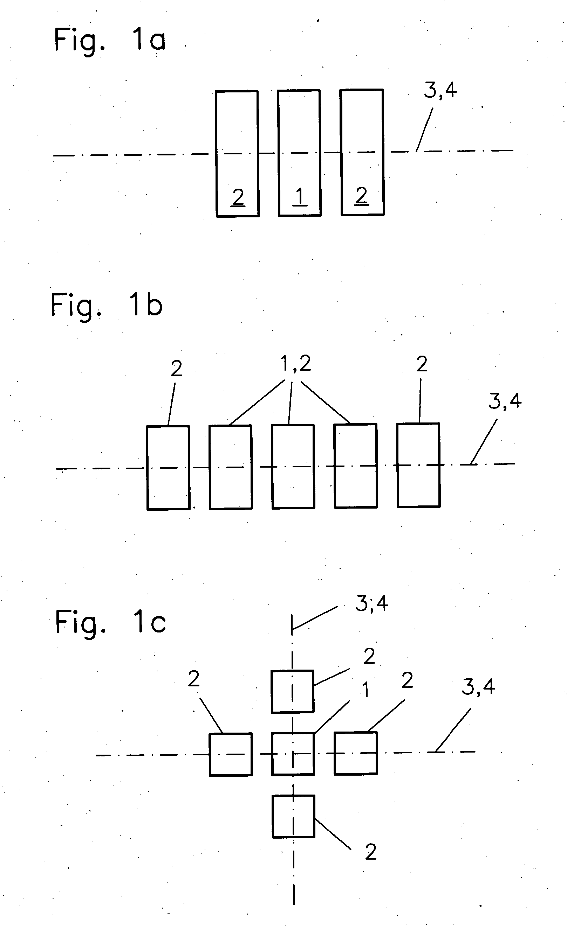

Wesentliche Elemente des Sensorsystems sind die Sensoren **17** mit einer grundlegenden Elektrodenanordnung, wie sie in **Fig.1a** **bis c** beispielhaft und nicht abschließend wiedergegeben sind.

Sie umfassen jeweils eine in **Fig.1a** dargestellte Anordnung mit einer mittleren Elektrode **1** (Bezugselektrode) sowie mindestens zwei um diese beidseitig in einer Linie angeordnete Messelektroden **2.** Die beidseitige Anordnung der beiden Messelektroden ist vorzugsweise symmetrisch zur mittleren Elektrode, wobei die Messelektroden vorzugsweise eine einheitliche Fläche aufspannen. Erstrecken sich Elektrode und Messelektroden achsensymmetrisch beidseitig um eine Symmetrieachse **3,** gibt diese die Ausrichtung **4** des Sensors vor.

Eine Ausführung des Sensors **17** sieht mehr als drei Elektroden vor, in **Fig.1b** beispielhaft dargestellt mit fünf hintereinander in einer Reihe um die Symmetrieachse angeordneten Elektroden. Dies ermöglicht eine freie Wahl der drei benachbarten Elektrodenflächen, umfassend eine mittlere Elektrode (Bezugspotential) und zwei beidseitig und in Ausrichtung angrenzende Messelektroden zur Erfassung der Potentialunterschiede. Diese Ausführung ermöglicht ferner einen Einsatz von mehr als zwei Messelektroden um die Bezugselektrode und auch in besonders vorteilhafter Weise auch eine im laufenden Betrieb wählbare Anzahl von Messelektroden oder ein Wechsel der Bezugselektrode von einer auf eine andere Elektrode. Vorzugsweise erfolgt die Auswahl der drei Elektroden durch eine Signalverarbeitung, wobei vorzugsweise eine größere Abweichung der beiden durch den Sensor erfassten Potentialunterschiede zueinander (z.B. durch Überschreitung eines programmierbaren Limits) eine Änderung der Auswahl auslöst. Dabei ist es vorteilhaft, wenn alle Elektroden sowohl eine einheitliche und um die Symmetrieachse symmetrische Fläche als auch einen gleichen Abstand zueinander aufweisen. Mit der in **Fig.1b** dargestellten Variante ist ein Weiterbetrieb des Sensors insbesondere bei System- und Messstörungen in vorteilhafter Weise allein durch eine andere Auswahl und/oder Ansteuerung der Elektroden als Bezugs- und Messelektrode und ohne Erfordernis einer Neuapplizierung realisierbar.

**Fig.1c** zeigt einen Sensor **17** mit kreuzweise um eine Bezugselektrode und zwei Symmetrieachsen angeordneten Messelektroden. Die beiden Symmetrieachsen geben die beiden Ausrichtungen des Sensors wieder.

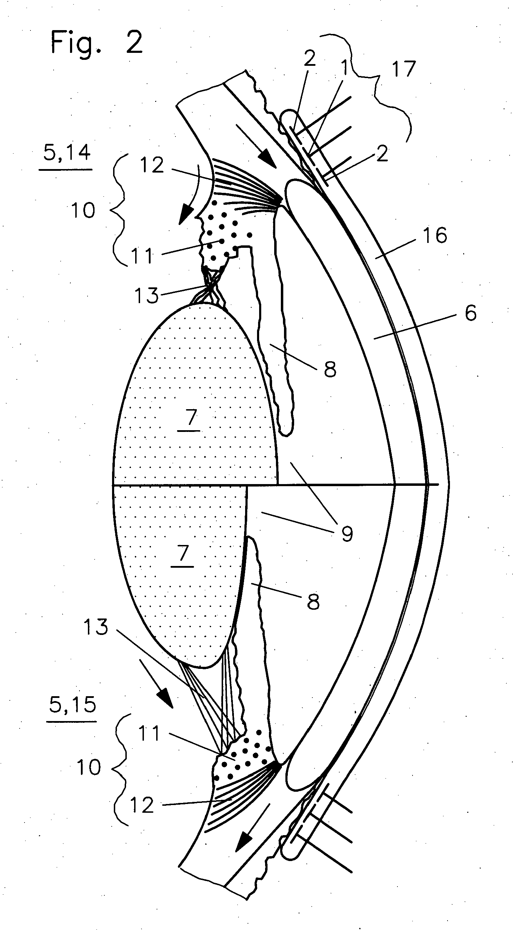

Eine schematische Schnittdarstellung eines Teils des Auges mit applizierten Sensorsystem mit Elektroden zeigt **Fig.2****.** Die Ansicht des Auges **5** erfasst die Cornea **6,** die Linse **7,** den Irismuskel **8,** die Pupille **9** sowie die Ziliarmuskel **10.** Der Ziliarmuskel teilt sich in den Müllerschen Muskel **11** und den Brückschen Muskel **12** auf. Die Darstellung des Augenabschnitts ist zweigeteilt, wobei die obere und untere Hälfte einen voneinander abweichenden Akkommodationszustand der natürlichen Optik darstellt. Deutlich ist die Verformbarkeit der Linse und dem mit dieser über Zenulafasern **13** verbundenen Ziliarmuskel zu erkennen; der obere Bereich **14** repräsentiert eine Nahsichteinstellung, der untere Bereich **15** eine Fernsichteinstellung.

Auf der Cornea **6** ist eine Kontaktlinse **16** als Kontaktelement und Träger für die Sensoren **17** aufgesetzt. Ein sich über dem Ziliarmuskel um die Iris bzw. der Pupille umlaufender Bereich des Kontaktelements ist die Ringfläche **18,** auf dem die Sensoren **17** mit direktem Kontakt zur Cornea **6** angeordnet sind. Die in **Fig.2** nicht näher dargestellte Signalverarbeitung besteht aus elektronischen Bauteilen und ist vorzugsweise auf der Ringfläche oder im Bereich über der Iris im Kontaktelement integriert. Der zentrale Bereich der Kontaktlinse über der Pupille umfasst ein vorzugsweise integriertes Linsensystem (in **Fig.2** nicht dargestellt), beispielsweise ein künstliches Akkommodationssystem mit über das Sensorsystem in der Brennweite einstellbarem Linsensystem.

Die Elektroden sind vorzugsweise am Rand der Kontaktlinse aufgebracht und nehmen dort Potentialänderungen durch Muskelaktivität auf. Am Kontaktlinsenrand ist der Abstand zum Ziliarmuskel, dessen Signal erfasst werden soll, besonders gering. Die Amplitude des Ziliarmuskelsignals ist deshalb deutlich größer, als Störsignale von anderen Muskeln (z.B. Irismuskeln), die weiter von den Elektroden entfernt liegen.

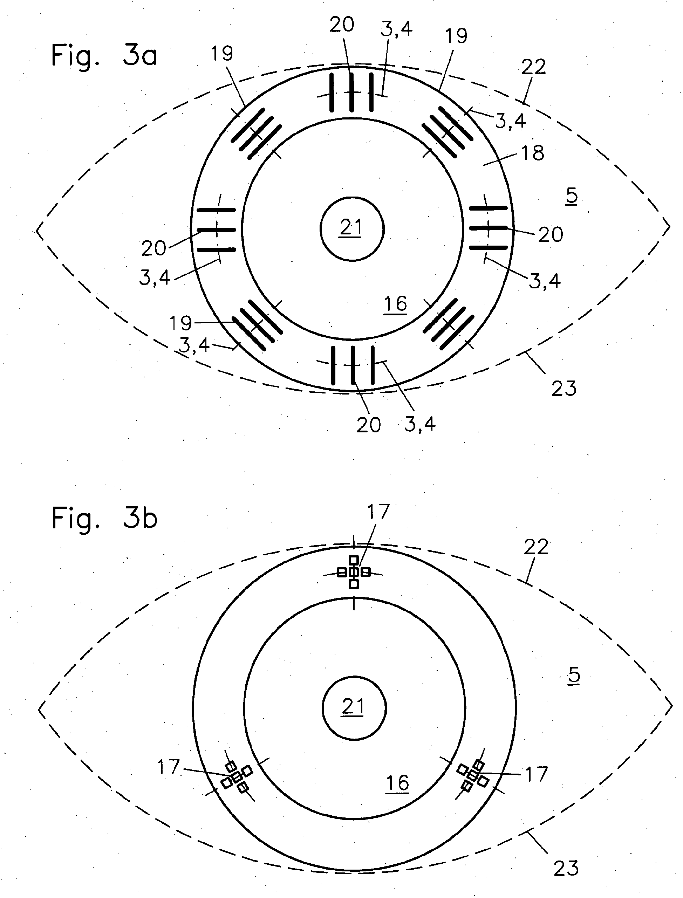

**Fig.3a** **bis d** offenbaren ausgewählte Ausführungsbeispiele der Sensoranordnung und -ausrichtung des Sensorsystems. Dargestellt ist eine frontale Ansicht der Kontaktlinse **16**, wobei das Auge **5** schematisch im Bereich zwischen dem oberen und unteren Augenlid **22** bzw. **23** angedeutet ist.

Eine besonders bevorzugte Ausführung gem. **Fig.3a** umfasst neben den vorzugsweise mindestens vier Sensoren mit zum zentralen Pupillenbereich **21** radialer Ausrichtung **19** mindestens vier Sensoren mit tangentialer Ausrichtung **20,** weiter bevorzugt wie dargestellt in mit der Ausrichtung abwechselnder Reihenfolge aufgereiht auf der Ringfläche **18** konzentrisch zur und um die Iris. Der Müllersche Muskel ist ein Ringmuskel, dessen Ansteuersignal an jeder Stelle der Ringfläche mit hinreichender Sicherheit erfassbar ist. Dagegen sind die Brückschen Muskel radial zur Iris angeordnet. Eine Muskelfaser dieser Muskel nur durch den direkt darüber angeordneten Sensor erfassbar ist, sodass diese jeweils nur an einer Stelle der Ringfläche detektierbar ist. Zudem sind die Müllersehen Muskeln innen am Ziliarkörper, die Brückschen Muskeln dagegen weiter außen am Ziliarkörper angeordnet. Durch die Lage der Müllersehen Muskeln am inneren Rand des Ziliarkörpers ist der Abstand der Müllersehen Muskeln zu den Sensorelektroden größer als bei den Brückschen Muskeln. Insbesondere zur Erhöhung des Signal-Rausch-Abstandes der tangentialen Elektroden ist es vorteilhaft, gerade die Sensoren mit tangentialer Ausrichtung mit einer größeren Anzahl redundant über die Ringfläche verteilt anzuordnen. Die Ausführung repräsentiert beispielhaft ein Sensorsystem, bei dem die Sensoren mindestens vier in mindestens zwei Ausrichtungen um die mittlere Elektrode angeordnete Elektroden umfassen, wobei die Ausrichtungen sowohl tangential und als auch radial zur Iris orientierte Ausrichtungen umfassen. Durch das Aufbringen von mehreren Messstellen kann das Signal-zu-Rausch Verhältnis (SNR) deutlich erhöht werden.

Eine Kombination von radial und tangential ausgerichteten Elektroden führt zu einer kreuzförmigen Anordnung der Elektroden der Sensoren (vgl. **Fig.1c****).** Vorzugsweise werden im Rahmen einer Ausführung mindestens drei Sensoren auf der Ringfläche um die Iris angeordnet **(****Fig.3b****).**

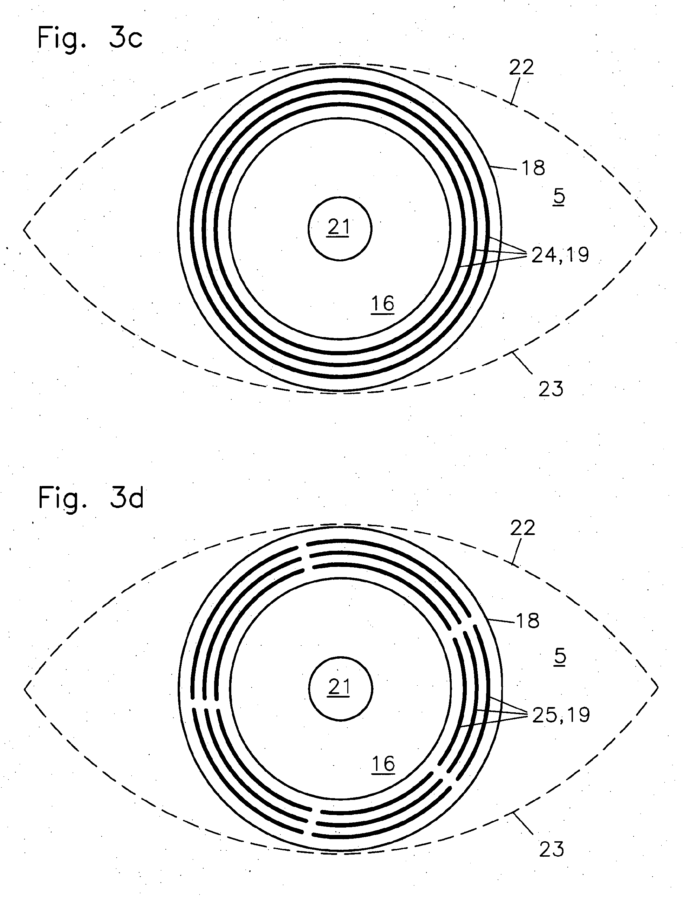

**Fig.3c** zeigt ein weiteres Ausführungsbeispiel mit einem Sensor **17,** der mindestens drei, vorzugsweise genau drei auf der Ringfläche konzentrisch zur Iris und vorzugsweise parallel zueinander angeordnete Elektrodenringe **24** als Elektroden umfasst. Eine mittlere Elektrode dient dabei als Masse (Referenzpotential). Die Elektrodenringe sind in einer weiter bevorzugten Ausführung als Ringe nicht unterbrochen. Die Ausrichtung dieses Sensors ist radial zum zentralen Pupillenbereich **21,** d.h. zur Pupille und zur Iris. Dieser Sensor erfasst damit Muskelfasern der Brückschen Muskeln jeden Winkelbereich der gesamten Ringfläche und ist besonders vorteilhaft bei bereits durch Krankheiten, Infarkte oder sonstigen Lähmungen geschädigten einzelner Brückschen Muskeln. Es genügt ein intakter Brückscher Muskel für die Erfassung der Ansteuersignale, der durch diese Ausführung in jeden Fall von der Elektrode überdeckt ist.

In einem weiteren Ausführungsbeispiel gem. **Fig.3d** sind die Ringelelektroden in Kreisbogenabschnitte **25,** die jeweils einen Winkelbereich um die Iris abdecken, unterteilt. Dies ermöglicht eine selektive Erfassung der Brückschen Muskeln je Winkelbereich und damit die Erkennung und Überwachung z.B. von sich ändernden pathologischen Zuständen wie Krankheits- oder Gesundungsverläufe in den jeweiligen Winkelbereichen, wobei in Summe jeder Winkelbereich abgedeckt ist. Das Sensorsystem ist optional mit Sensoren mit tangentialer Ausrichtung allerdings auf einer eigenen zweiten Ringfläche auf dem Kontaktelement kombinierbar. Durch Vergleichsmessungen benachbarter Sensoren sind zudem Unterschiede in der Ansteuerung der Brückschen Muskeln oder auch ein Verschieben des Kontaktelements auf dem Auge erfassbar.

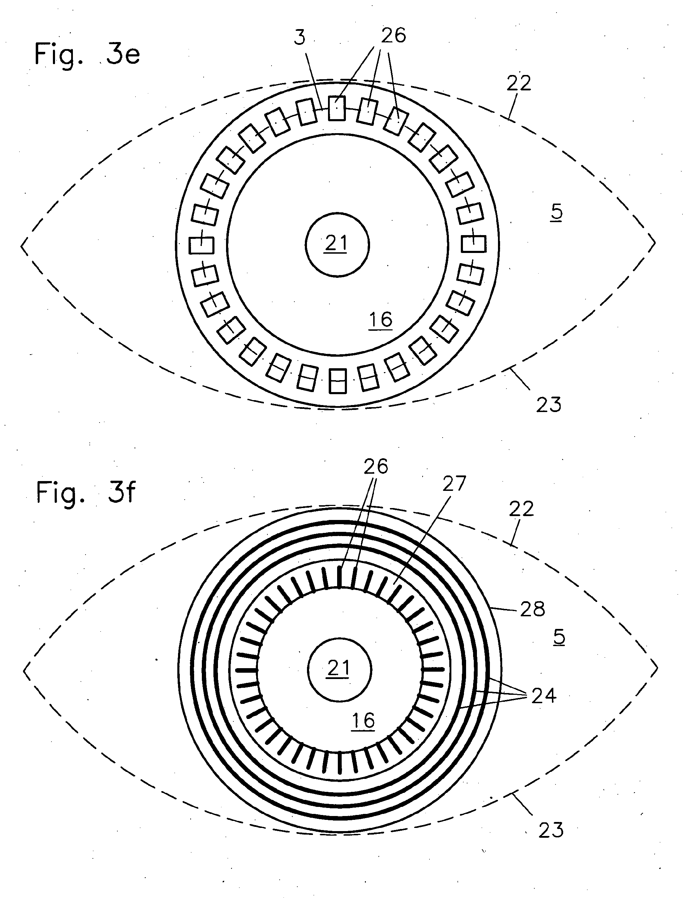

Eine Ausgestaltung hierzu sieht vor, einen vorzugsweise umlaufenden Elektrodenring mit einer Vielzahl von Einzelelektroden **26** mit tangentialer Symmetrieachse **3** und damit Ausrichtung (nicht erfindungsgemäße **Fig.3e****).** Vorzugsweise sind alle Elektrodenflächen mit der Signalverarbeitung fest verdrahtet. Bei dieser Ausführung sind je nach Bedarf je drei beliebige, aber benachbarte Elektroden als Elektrodengruppe zu einem Sensor schaltbar, und zwar in vorteilhafter Weise ohne eine Änderung der Anordnung allein durch eine individuelle Ansteuerung der Elektrodenflächen durch die Signalverarbeitung. Eine Variante sieht vor, mindestens drei Eletrodengruppen über den Umfang des Elektrodenrings vorzugsweise in gleichem Abstand zueinander simultan zu aktivieren. Hierdurch werden die Ansteuersignale der Müllersche Muskel an drei Stellen simultan erfasst, wobei die Werte in der Signalverarbeitung nach optionaler Eliminierung von Fehlmessungen vorzugsweise gemittelt werden. Fehlmessungen veranlassen die Signalverarbeitung vorzugsweise zu einer Auswahl von neuen Elektrodengruppen an einer anderen Stelle, d.h. eine Verschiebung des Sensors über den Müllerschen Muskeln.

Eine weitere Ausführung sieht vor die Ringfläche in zwei oder mehrere Teilringflächen zu unterteilen, wobei jede Ringfläche vorzugsweise (vgl. **Fig.3f****),** aber nicht zwingend mit Sensoren nur einer Ausrichtung bestückt ist. Wie als Beispiel dargestellt befinden sich beispielsweise auf der inneren Teilringfläche **27** ein umlaufenden Elektrodenring mit einer Vielzahl von Einzelelektroden und umlaufender Symmetrieachse (vgl. **Fig.3e****)** zur Erfassung des Müllerschen Muskels und auf der äußeren Teilringfläche **28** Ringelektroden zur Erfassung der Brückschen Muskel (vgl. **Fig.3c** **oder d).**

Grundsätzlich werden die Elektroden in ihrer Symmetrieachse vorzugsweise radial oder tangential ausgerichtet angeordnet. Bei den radial ausgerichteten Elektroden werden vornehmlich Signale detektiert, die von den radial verlaufenden Muskelfasern (Brücksche Muskel) stammen. Es ist zu erwarten, dass im Radialsignal auch ein Anteil der Irisaktivität zu erkennen ist. Die tangentiale Anordnung der Elektroden führt hingegen zur vornehmlichen Detektion der von tangential verlaufenden Muskelfasern (Müllersche Muskel) stammenden Teile des Ziliarkörpers. Dabei werden Einflüsse der Irismuskulatur stark reduziert. Zur Glättung des Referenzpotentials können alle Referenzelektroden der verschiedenen Messstellen miteinander verbunden werden. Insofern ist es vorteilhaft, dass die radial ausgerichteten Elektroden auf den äußeren und die tangential ausgerichteten Elektroden vorzugsweise auf den inneren Teilringflächen angeordnet sind. Diese Anordnung kommt auch der Anatomie des Auges entgegen, da die Müllerschen Muskeln wie die tangentialen Elektroden weiter innen und die Brückschen Muskeln sowie die radialen Elektroden außen angeordnet sind.

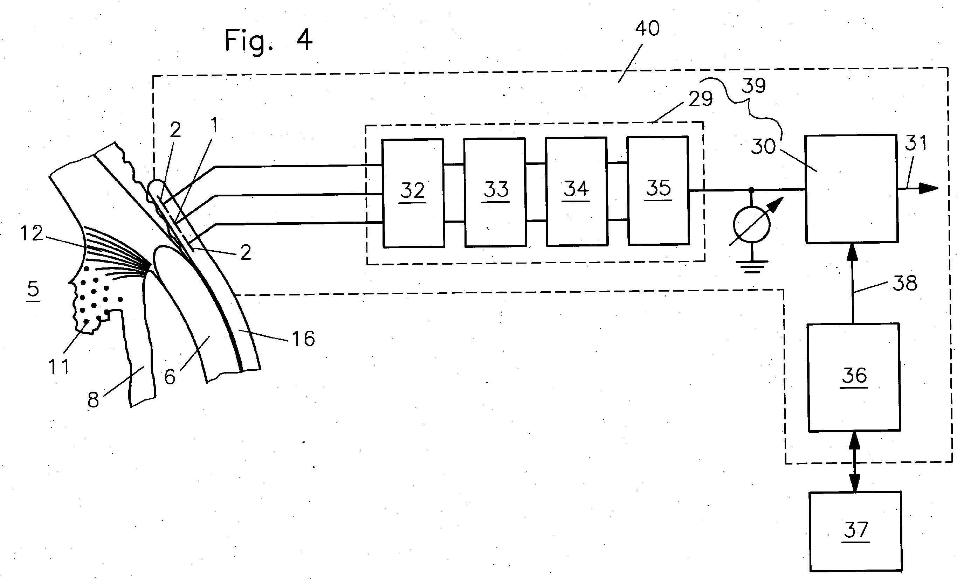

Das Sensorsystem **40** umfasst ferner eine Signalverarbeitung **39** mit einer Auswerteelektronik **(****Fig.4****),** die in vorgenannter Weise vorzugsweise mit auf der Kontaktlinse integriert ist. Die Signalverarbeitung umfasst eine Signalkonditionierung **29** sowie eine Auswerteeinheit **30** mit Signalerkennung und Generator für ein Steuersignal **31.** Nicht weiter dargestellt ist eine Energiequelle des Sensorsystems, beispielsweise ein kapazitiver Energiepuffer der über Bewegungen des Systems im Erdmagnetfeld, durch Trägheitsgeneratoren oder Lidschlagsgeneratoren gespeist wird. Alternativ ist eine kontaktlose Übertragung der erforderlichen Energie von außerhalb denkbar, beispielsweise über elektromagnetische Übertragungsmittel. Weiterhin sind Solarzellen, die das Umgebungslicht in elektrische Energie wandeln oder Bio-Brennstoffzellen einsetzbar, die die chemische Energie der Glukose der Tränenflüssigkeit in elektrische Energie wandeln.

Die Auswerteelektronik soll die Potentialdifferenz geringer Amplitude zwischen den Elektroden so aufbereiten, dass eine Berechnung des Akkommodationsbedarfs möglich wird. Die an die mittlere Elektrode **1** (Bezugspotential, z.B. auf Masse gelegt) angrenzenden Messelektroden **2** der Sensoren sind über elektrische Leiter, vorzugsweise Leiterbahnen mit der Signalkonditionierung **29** verbunden und geben das Messsignal in dieser zunächst an eine ESD-Schutzschaltung **32** weiter. Damit wird beim üblichen Umgang (Einsetzen, Entfernen, Reinigen) des Benutzers mit der Kontaktlinse ein Defekt der Elektronik durch elektrostatische Entladung verhindert. Eine ESD-Schutzvorrichtung schützt die Auswerteeinheit insbesondere bei der Handhabung durch den Systemträger beim Einsetzen, Entfernen und Reinigen des Systems vor auftretenden Überspannungen. Es folgt eine ein- oder mehrstufige Verstärkerschaltung **33,** um die Amplitude des Signals zu erhöhen. Bei einer bi- oder multipolaren Konfiguration können Gleichtaktstörspannungen durch eine Differenzstufe unterdrückt werden. Diese wird bevorzugt mit einem Instrumentenverstärker realisiert. Anschließend wird das Signal in Filter **34** gefiltert. Störungen und hochfrequentes Rauschen werden in bevorzugt mehreren Filterstufen unterdrückt. Es folgt eine Gleichrichterschaltung **35,** in der das Wechselspannungssignal in eine Gleichspannung gewandelt wird, die proportional zur Muskelaktivität ist. Um sowohl die positiven als auch negativen Signalanteile zur Detektion nutzen zu können, wird der Einsatz eines Vollweggleichrichters bevorzugt. Das aufbereitete gleichgerichtete Messsignal wird an die Auswerteeinheit weitergeleitet.

Eine Mittelung oder Gewichtung der vorzugswiese zwei in einem Sensor erfassten Spannungen kann optional in der Auswerteeinheit beispielsweise durch einen Subtrahierer und/oder Filter, der die bereits aufbereiteten Signale bestimmter Elektroden verarbeitet, erfolgen. Beispielsweise werden insbesondere Messsignale im Randbereich zu einem benachbarten Muskel, der nicht erfasst werden soll, durch diesen dennoch beeinflusst. Dieser Störeinfluss lässt sich durch einen Vergleich der redundant über mehrere Elektroden eines Sensors erfassten Messsignale (in der Regel zwei) erkennen und in der Auswerteeinheit eliminieren. Hierdurch kann auch eine bessere Separation tangentialer und radialer Muskelsignale erreicht werden und beispielsweise das Irissignal (radial) im tangentialen Ziliarmuskelsignal unterdrückt werden.

Die Auswerteeinheit **30** leitet aus der Höhe des aufbereiteten gleichgerichteten Messsignals relativ zum Bezugspotential den Akkommodationsbedarf ab und generiert das Steuersignal **31.** Bei Anordnungen mit mehreren Messstellen erfolgt vorzugsweise zusätzlich eine Mittelung oder Gewichtung der Messignale, um Auswirkungen von übersprechenden, unerwünschten Muskelsignalen (z.B. Irismuskulatur) auf die Auswertung zu reduzieren.

Das Steuersignal **31 dient** der Einstellung einer Brennweite eines verstellbaren Linsensystems und wird über Übertragungsmittel (Leiterbahn oder berührungslos über elektromagnetische Wellen) an dieses weitergeleitet. Alternativ lässt sich das vorgenannte aufbereitete gleichgerichtete Messsignal durch Übertragungsmittel der vorgenannten Art berührungslos z.B. an eine zentrale Auswerteeinheit für beide Augen weiterleiten.

Optional kann eine Offset-Korrektur und Kalibrierung durchgeführt werden. Über eine Kommunikationsschnittstelle **36** zu einem externen Computer **37** werden hierzu benutzerspezifische Kalibrierungsdaten **38** in die Auswerteeinheit **30** übertragen. Somit kann das System schnell auf jeden einzelnen Benutzer optimal angepasst werden.

### Literatur:

[1] Strenk B. et al.: Age-related changes in human ciliary muscle and lens: A magnetic resonance imaging study; Investigative Ophthalmology and Visual Sci.40 (1999) 6 S.1162-1169
[2] EP 1 919 360 B1
[3] Klink S.: Neues System zur Erfassung des Akkommodationsbedarfs im menschlichen Auge; Schriftenreihe des Instituts für angewandte Informatik/Automatisierungstechnik Universität Karlsruhe (TH) Band 23, Universitätsverlag Karlsruhe 2008, ISBN 978-3-86644-300-6
[4] US 4.386.831
[5] RU 2 281 020 C1
[6] US 7.404.636 B2
[7] US 6.851.805 B2
[8] DE 10 2005 038 542 A1

### Bezugszeichenliste

- 1: mittlere Elektrode
- 2: Messelektrode
- 3: Symmetrieachse
- 4: Ausrichtung
- 5: Auge
- 6: Cornea
- 7: Linse
- 8: Irismuskel
- 9: Pupille
- 10: Ziliarmuskel
- 11: Müllersche Muskel
- 12: Brücksche Muskel
- 13: Zenularfasern
- 14: obere Bereich
- 15: untere Bereich
- 16: Kontaktlinse
- 17: Sensor
- 18: Ringfläche
- 19: Sensor mit radialer Ausrichtung
- 20: Sensor mit tangentialer Ausrichtung
- 21: Pupillenbereich.
- 22: obere Augenlid
- 23: untere Augenlid
- 24: Elektrodenringe
- 25: Kreisbogenabschnitt
- 26: Einzelelektrode
- 27: innere Teilringfläche
- 28: äußere Teilringfläche
- 29: Signalkonditionierung
- 30: Auswerteeinheit
- 31: Steuersignal
- 32: ESD-Schutzschaltung
- 33: Verstärkerschaltung
- 34: Filter
- 35: Gleichrichterschaltung
- 36: Kommunikationsschnittstelle
- 37: Computer
- 38: Kalibrierungsdaten
- 39: Signalverarbeitung
- 40: Sensorsystem

## Patentansprüche

1. Sensorsystem für die Erfassung der Ansteuersignale eines Ziliarmuskels **(10)** eines Auges **(5),** umfassend
a) ein auf der Cornea **(6)** um die Iris **(8)** eines Auges applizierbares Kontaktelement **(16)** aus einem elektrisch nicht leitenden Werkstoff,
b) mindestens einen am Kontaktelement aufgebrachten Sensor **(17)** mit ringförmiger Anordnung um die Iris sowie
c) eine Signalverarbeitung **(39)** auf oder im Kontaktelement, wobei
d) jeder Sensor mindestens drei in mindestens einer Ausrichtung **(4)** in Reihe angeordnete Elektroden **(1, 2)** umfasst,
e) die mindestens eine Ausrichtung eine radial zur Iris orientierte Ausrichtung umfasst sowie
f) eine jeweils mittlere Elektrode **(1)** eines jeden Sensors gegenüber den in einer Ausrichtung zwei unmittelbar angrenzenden Elektroden **(2),** die das Körperpotential am Augapfel abgreifen, ein Bezugspotential bildet.

2. Sensorsystem nach Anspruch 1, **dadurch gekennzeichnet, dass** der Sensor **(17)** oder die Sensoren auf einer Ringfläche **(18)** um die Iris angeordnet sind.

3. Sensorsystem nach Anspruch 1 oder 2, **dadurch gekennzeichnet, dass** mindestens zwei Sensoren **(17)** um die Iris angeordnet sind, deren Ausrichtungen **(4)** sowohl tangential und als auch radial zur Iris orientierte Ausrichtungen umfassen.

4. Sensorsystem nach Anspruch 3, dadurch gekenntzeichnet, dass die mindestens zwei Sensoren **(17)** entweder nur eine tangential oder nur eine radial zur Iris orientierte Ausrichtung aufweisen und auf der Ringfläche **(18)** in abwechselnder Ausrichtung um die Iris aufgereiht sind.

5. Sensorsystem nach einem der vorgenannten Ansprüche 1 bis 3, **dadurch gekennzeichnet, dass** die Sensoren mindestens vier in mindestens zwei Ausrichtungen um die mittlere Elektrode **(1)** angeordnete Ektroden **(2)** umfassen, wobei die Ausrichtungen **(4)** sowohl tangential und als auch radial zur Iris orientierte Ausrichtungen umfassen.

6. Sensorsystem nach Anspruch 2, **dadurch gekennzeichnet, dass** ein Sensor drei auf der Ringfläche konzentrisch zur Iris angeordnete Elektrodenringe **(24)** oder Elektrodenringabschnitte **(25)** als Elektroden umfasst.

7. Sensorsystem nach einem der vorgenannten Ansprüche, **dadurch gekennzeichnet, dass** mindestens vier Sensoren **(17)** mit tangentialer Ausrichtung um die Iris angeordnet sind.

8. Sensorsystem nach einem der vorgenannten Ansprüche, **dadurch gekennzeichnet, dass** das Kontaktelement **(16)** eine Kontaktlinse ist.

9. Sensorsystem nach einem der vorgenannten Ansprüche, **dadurch gekennzeichnet, dass** die Signalverarbeitung **(39)** eine Signalkonditionierung **(29),** eine Signalerkennung **(30)** mit einem Generator für ein Steuersignal umfasst.

10. Sensorsystem nach Anspruch 9, **dadurch gekennzeichnet, dass** die Signalverarbeitung **(39)** Übertragungsmittel zu einem durch das Steuersignal in seiner Brennweite aktiv verstellbaren Linsenkörper oder Linsensystem umfasst.

## Claims

1. Sensor system for detecting the activation signals of a ciliary muscle (10) of an eye (5), comprising
a) a contact element (16) which can be applied to the cornea (6) around the iris (8) of an eye wherein the contact element (16) is made of an electrically non-conductive material,
b) at least one sensor (17) located at the contact element, with an annular arrangement around the iris, and
c) a signal processing unit (39) on, or in, the contact element, wherein
d) each sensor comprises at least three electrodes (1, 2) arranged in a row in at least one alignment (4),
e) the at least one alignment comprises an alignment oriented radially to the iris, and
f) an electrode (1) in each case located in the middle of each sensor, opposite the two immediately adjacent electrodes (2) in an alignment, which detects the body potential at the eyeball and forms a reference potential.

2. Sensor system according to claim 1, **characterised in that** the sensor (17) or the sensors are arranged on a ring surface (18) around the iris.

3. Sensor system according to claim 1 or 2, **characterised in that** at least two sensors (17) are arranged around the iris, the alignments (4) of which comprise alignments which are oriented both tangentially as well as radially to the iris.

4. Sensor system according to claim 3, **characterised in that** the at least two sensors (17) exhibit either only one alignment oriented tangentially to the iris or only one oriented radially to the iris, and are arrayed on the ring surface (18) in alternating alignment around the iris.

5. Sensor system according to any one of the preceding claims 1 to 3, **characterised in that** the sensors comprise at least four electrodes (2) arranged in at least two alignments about the middle electrode (1), wherein the alignments (4) exhibit alignments oriented both tangentially as well as radially to the iris.

6. Sensor system according to claim 2, **characterised in that** one sensor comprises three electrode rings (24) or electrode ring sections (25) as electrodes arranged on the ring surface concentrically to the iris.

7. Sensor system according to any one of the preceding claims, **characterised in that** at least four sensors (17) are arranged with tangential alignments around the iris.

8. Sensor system according to any one of the preceding claims, **characterised in that** the contact element (16) is a contact lens.

9. Sensor system according to any one of the preceding claims, **characterised in that** the signal processing unit (39) comprises a signal conditioning element (29), a signal recognition element (30) with a generator for a control signal.

10. Sensor system according to claim 9, **characterised in that** the signal processing unit (39) comprises transfer means to a lens body or lens system which can be actively adjusted in its focal length by the control signal.

## Revendications

1. Système de capteur permettant la détection des signaux de commande d'un muscle ciliaire (10) d'un oeil (5) comprenant :
a) un élément de contact (16) en un matériau non électriquement conducteur pouvant être appliqué sur la cornée (6) autour de l'iris (8) d'un oeil,
b) au moins un capteur (17) appliqué sur l'élément de contact avec un positionnement annulaire autour de l'iris, et
c) un dispositif de traitement de signal (39) situé sur ou dans l'élément de contact,
d) chaque capteur comprenant au moins trois électrodes (1, 2) montées en série dans au moins une direction (4),
e) la direction comporte une direction orientée radialement par rapport à l'iris, et
f) une électrode respectivement centrale (1) de l'un des capteurs formant, un potentiel de référence par rapport aux deux électrodes immédiatement voisines (2) dans une direction qui palpent le potentiel corporel au niveau du globe oculaire.

2. Système de capteur conforme à la revendication 1,
**caractérisé en ce que**
le ou les capteur(s) (17) est(sont) situé(s) sur une surface annulaire (18) autour de l'iris.

3. Système de capteur conforme à la revendication 1 ou 2,
**caractérisé en ce qu'**
autour de l'iris sont positionnés au moins deux capteurs (17) dont les directions (4) comportent des directions orientées tangentiellement et également radialement par rapport à l'iris.

4. Système de capteurs conforme à la revendication 3,
**caractérisé en ce que**
les capteurs (17) qui sont au moins au nombre de deux ne comportent qu'une seule direction orientée tangentiellement par rapport à l'iris, ou qu'une seule direction orientée radialement par rapport à l'iris, et sont positionnés en rangées sur la surface annulaire (18) autour de l'iris avec des directions alternées.

5. Système de capteur conforme à l'une des revendications précédentes 1 à 3,
**caractérisé en ce que**
les capteurs comportent au moins quatre électrodes (2) positionnées dans au moins deux directions autour de l'électrode centrale (1), les directions (4) comprenant des directions orientées tangentiellement ainsi que des directions orientées radialement par rapport à l'iris.

6. Système de capteur conforme à la revendication 2,
**caractérisé en ce qu'**
un capteur comporte en tant qu'électrodes trois anneaux d'électrodes (24) ou segments annulaires d'électrodes (25) positionnés concentriquement par rapport à l'iris.

7. Système de capteur conforme à l'une des revendications précédentes,
**caractérisé en ce qu'**
au moins quatre capteurs (17) sont positionnés autour de l'iris avec des directions tangentielles.

8. Système de capteur conforme à l'une des revendications précédentes,
**caractérisé en ce que**
l'élément de contact (16) est une lentille de contact.

9. Système de capteur conforme à l'une des revendications précédentes,
**caractérisé en ce que**
le dispositif de traitement de signal (39) comprend un dispositif de conditionnement de signal (29), un dispositif de reconnaissance de signal (30) avec un générateur d'un signal de commande.

10. Système de capteur conforme à la revendication 9,
**caractérisé en ce que**
le dispositif de traitement de signal (39) comprend des moyens de transmission à un corps de lentilles ou à un système de lentilles dont la distance focale peut être réglée activement par le signal de commande.
